# EUROPEAN PATENT SPECIFICATION

(11) **EP 3 456 312 B1**
(45) Date of publication and mention of the grant of the patent: **18.06.2025**
(21) Application number: 16901173.1
(22) Date of filing: 10.11.2016
(51) Int. Cl.: A61K 8/97, A61K 8/92, A61K 8/73, A61Q 19/00, A61K 8/60, A61K 8/9706, A61K 8/9783, A61Q 19/08

(54) **PROBIOTIC COSMETIC COMPOSITIONS AND USE OF THE PROBIOTIC COSMETIC COMPOSITIONS**
PROBIOTISCHE KOSMETISCHE ZUSAMMENSETZUNGEN UND VERWENDUNG DER PROBIOTISCHEN KOSMETISCHEN ZUSAMMENSETZUNGEN
COMPOSITIONS COSMÉTIQUES PROBIOTIQUES ET UTILISATION DE CES COMPOSITIONS COSMÉTIQUES PROBIOTIQUES

(30) Priority: 12.05.2016 US 201662335381 P
(43) Date of publication of application: 20.03.2019
(73) Proprietor: Natura Cosméticos S.A., 05106-000 São Paulo SP (BR)
(72) Inventor: DE MIRANDA CHAVES VASQUEZ PINTO, Luciana, 05106-000 São Paulo - SP (BR); DOMENES PALMIERI RODRIGUEZ, Débora, 05106-000 São Paulo - SP (BR)
(74) Representative: Simmons & Simmons
(86) International application number: PCT/BR2016/050293
(87) International publication number: WO 2017/193185

(56) References cited:
- EP-A1- 2 671 615
- WO-A2-2015/031971
- GB-A- 2 498 543
- US-A- 5 601 833
- DATABASE GNPD [online] MINTEL; 24 July 2015 (2015-07-24), ANONYMOUS: "Repairing Smoothing Cream", XP055638736, retrieved from www.gnpd.com Database accession no. 3325039
- DATABASE GNPD [online] MINTEL; 4 December 2006 (2006-12-04), ANONYMOUS: "Buriti & Vegetal Protein Restorative Hair Mask", XP055638735, retrieved from www.gnpd.com Database accession no. 624732
- DATABASE GNPD [online] MINTEL; 9 March 2016 (2016-03-09), ANONYMOUS: "Night Recovery Moisturizer for Combination Oily to Oily Skin", XP055639012, retrieved from www.gnpd.com Database accession no. 3757405
- DATABASE GNPD [online] MINTEL; 15 April 2016 (2016-04-15), ANONYMOUS: "Rejuvenating Cream", XP055639016, retrieved from www.gnpd.com Database accession no. 3651017
- DATABASE GNPD [online] MINTEL; 28 October 2015 (2015-10-28), ANONYMOUS: "Regenerative Night Cream", XP055639023, retrieved from www.gnpd.com Database accession no. 3457917
- DATABASE GNPD [online] MINTEL; 26 June 2015 (2015-06-26), ANONYMOUS: "Exfoliant Bar Soap", XP093009010, retrieved from https://www.gnpd.com/sinatra/recordpage/3269359/ Database accession no. 3269359
- DATABASE GNPD [online] MINTEL; 3 March 2016 (2016-03-03), ANONYMOUS: "Advanced Wrinkle Fighter 360° Eye Serum", XP093009025, retrieved from https://www.gnpd.com/sinatra/recordpage/3772771/ Database accession no. 3772771

## Description

### FIELD OF THE INVENTION

The present invention relates to prebiotic cosmetic compositions comprising at least one ingredient as defined in claims with prebiotic activity selected from sugars, oils and extracts for the treatment of signs of aging on the skin, fostering well-balanced skin, nourishing beneficial micro-organisms in the skin microbiota, protecting it from harsh environmental aspects, strengthening it, reducing sensitivity and irritations, and improving its defense system.

### PRIOR ART

Microbiota constitute the set of micro-organisms found in a specific system, such as the skin, for example. Skin microbiota consists mainly of bacteria, but also include viruses, fungi and protozoa.

Micro-organisms found in skin microbiota are defined by environmental conditions such as anatomical location, question, oil and sweat production, oxygen concentration and pH levels, among others, in addition to biological factors such as hormone swings and a person's age.

Microbiota is responsible for several physiological functions, such as the production of nutrients or protective peptides, as well as immunological response modulation.

Micro-organisms can cling to the skin by bonding to skin structures, such as carbohydrates or cell receptors in the skin cells, or may be organized into colonies held together by biofilm.

The biofilm forms a microenvironment with pH, oxygen concentration, nutrients and metabolites that are different from the skin. It also facilitates bacterial communication through quorum sensing and protects microbiota from endogenous and exogenous antimicrobial agents in the person's immunological system.

Human skin has approximately 1000 bacteria for each cell. Genetic sequencing studies show that there are bacteria not only on the skin surface, but also in the sebaceous and sweat glands, follicles and skin folds, as well as at even deeper skin levels such as the dermis.

The most common genera on the surface levels of the skin are *Staphylococcus, Propionibacterium, Micrococcus* and *Corynebacterium.*

These bacteria are distributed in different parts of the skin, depending on the microenvironment. For example, *Staphylococcus* and *Corynebacterium* are the most abundant genera on the skin, and remain there for life. *Propionibacterium,* which is anaerobic, is found in the sebaceous glands, while *Corynebacterium* is found in the armpits, and are related to the production of unpleasant smells. Found on the scalp, *Malassezia* is related to seborrheic dermatitis and dandruff.

In general, human skin offers a dry and mildly acid environment for microbiota, with only proteins and lipids available.

Keeping skin biota in an even balance involves adhesion mechanisms, interactions with the person's immunological defense system, alterations to the microenvironment, nutrient consumption and the production of antimicrobial agents

The surface layers of the skin are constantly flaking off, removing part of the microbiota. The capacity to adhere viable skin cells ensures that micro-organisms remain in place. Bacteria bond to membrane receptors in the cells, such as those in the toll-like family that recognize patterns associated with the bacteria (TLR 1-10), mannose receptors or NOD-like receptors. These receptors activate immunological pathways that distinguish pathogenic bacteria from their beneficial counterparts, stimulating appropriate responses. The microbiota composition teaches the immune system to distinguish beneficial bacteria more accurately, modulating inflammatory responses and reducing skin sensitivity to external factors such as variations in temperature, moisture and exposure to UV radiation.

Furthermore, microbiota produce lactic acid that, together with free fatty acids (from sebum), acid amino acids (from sweat) and urocanic and pyrrolidone carboxylic acids (from the corneocyte keratinization process), keeps the skin pH more acid and restricts micro-organisms in the biota. Lipids are the main source of carbon used by skin microbiota.

Variations in age, gender and hormone cycles interfere in the availability of nutrients for microbiota, and may result in micro-organism composition swings.

The role played by the skin, serving as a selective barrier against the outside environment, is facilitated considerably by antimicrobial peptides (AMPs) produced by keratinocytes and skin microbiota. More than 200 molecules with antimicrobial activities are described, produced by micro-organisms and cataloged in public and private databases. Among them are α and β-defensins (hBD), cathelicidins, C-type lectins, dermicidins, etc. Most AMPs consist of small peptides measuring approximately 2 to 15kDa. An imbalance (to many or too few) AMPs on the skin is related to pathologies such as atopical dermatitis, rosacea and psoriasis. Some factors produced by the skin, such as vitamin D and PTH, regulate AMP synthesis. These AMPs are important for protecting the skin against pathogens, controlling the microbiota population and limiting contact between keratinocytes and microbiota. The activity of the AMPs are generally broad-spectrum, encompassing Gram-positive and Gram-negative bacteria, as well as fungi, protozoa and viruses, in some cases.

Microbiota also produce other substances such hyaluronic acid produced by *Streptococcus* and some *Lactobacillus,* which is a constituent element of the extracellular matrix that fills the dermis, giving shape to the skin and stimulating the production of β-defensin 2 (hBD2).

Sphingomyelinase is produced by keratinocytes, *Streptococcus* and *Lactobacillus,* being an enzyme that participates in the synthesis of ceramides that are vital for skin barrier maintenance.

Lipoteichoic acid (LTA) is the structural component of Gram-positive bacteria such as *Staphylococcus, Lactobacillus* and *Bifidobacteria,* stimulating the production of antimicrobial peptides such as β-defensins (hBD) and cathelicidins.

Peptidoglycans are structural components of the cell walls of gram-positive bacteria, which modulate individual immunological responses, stimulating the production of IL-8 by keratinocytes and summoning phagocytes to infected sites.

Lactic acid (alpha hydroxy acid) produced by *Lactobacillus* and *Bifidobacteria* stimulates the skin shedding process, weakening cell adhesion and stimulating the production of ceramides by keratinocytes, thus strengthening the skin barrier and constituting a Natural Moisturizing Factor (NMF), which keeps moisture in the skin.

Produced by *Lactobacillus* and *Bifidobacteria,* acetic acid offers antibacterial activity against S. *aureus* and *P. aeruginosa.*

The main representatives of skin microbiota include *Staphylococcus epidermidis,* which is a major skin component, halotolerant and able to grow in environments with little water activity. Although generally harmless for the skin, this may cause nosocomial infections through colonizing foreign bodies such as catheters and implants in predisposed and immunocompromised people. Strongly AMP-resistant, they do not damage keratinocytes and produce AMPs (epidermin, Pep5, epilancin K7 and epicidin 280) that control S. *aureus* and *Streptococcus* populations.

However, the removal of S. *epidermidis* from the skin - through the excessive use of topical antibiotics, for example - leaves a person immunocompromised and very susceptible to infection by pathogens.

Furthermore, they synthesize proteases, keratinases, lipases and nucleases that are involved in the physiological turnover of the skin. They synthesize phenol-soluble modulins (PSMs), proteins with surfactant and selective antibacterial properties for S. *aureus* and *Streptococcus* through dissolving the biofilm formed by these pathogenic bacteria.

Staphylococcus aureus is found on healthy skins. The main human skin pathogen, it causes folliculitis, boils, subcutaneous abscesses, meningitis, endocarditis and septicemia. Conditions such as atopical dermatitis, virus (Herpes simplex type I or papillomavirus) and opportunistic yeast infections (*Trichophyton rubrum*) the skin more vulnerable to S. *aureus* infections.

The steady increase in the number of strains resistant to antibiotics such as metacyclin and vancomycin means that S. *aureus* has become a serious problem in hospital environments. It synthesizes bacteriocins such as staphylococcin 462 that inhibit other *S. aureus* strains.

Present on healthy skins, *Corynebacterium jeikeium* and *Corynebacterium striatum* are the most common micro-organisms, after S. *epidermidis.* They are found in the axillary, inguinal and perineal region, due to their high tolerance of salt concentrations. Adhering to keratinocytes at an estimated proportion of 25 bacteria/cell through fibronectin receptors, they produce bacteriocins such as lacticidin Q, which triggers the synthesis of dermicidin and cathelicidin in sweat, constituting a skin defense activity

Found in healthy microbiota on areas such as the face and back, *Propionibacteria acnes,* is an anaerobic saprophytic micro-organism found mainly in the sebaceous glands and associated with conditions such as folliculitis and acne vulgaris. The main source of its energy consists of lipids and fatty acids in sebum. The presence of porphyrin leaves P. acnes quite sensitive to UV radiation and stimulates the production of IL-8 and IL-12 by keratinocytes, forming a microenvironment that fosters inflammation.

Studies indicate that P. acnes is involved in the hypercornification process of the pilosebaceous duct, exacerbating inflammatory processes. Furthermore, it synthesizes acetic acid and propionic acid, in addition to lipases and proteases. It also synthesizes several bacteriocins such as propionicin PLG-1, jensenin G, propionicin SM1, SM2, T1 and acnein, being active against several lactic and Gram-negative bacteria, fungi and molds.

Malassezia furfur is a fastidious fungus found in healthy microbiota in regions such as the scalp and back, and is the main cause of opportunistic infections such as seborrheic dermatitis, dandruff and tinea versicolor (or pityriasis). Its main carbon source consists of free fatty acids. Furthermore, it synthesizes an enzyme that can degrade micro-organism cell walls, helping protect the skin against pathogens.

Microbiota imbalance may lead to seborrheic dermatitis, acne vulgaris and atopical dermatitis, among other diseases.

Skin microbiota is constantly improved through natural shedding and renewal of the skin. The capacity of micro-organisms to adhere to viable skin cells - including at deeper layers such as the dermis - is thus a competitive advantage.

The presence of microbiota modulates the inflammatory response of the skin, reducing sensitivity to factors such as temperature variations, moisture and exposure to UV radiation.

Microbiota produce lactic acid, which helps keep the skin pH more acid, antimicrobial peptides (AMPs) that protect the skin from pathogens and whose imbalances related to pathologies such as dermatitis, rosacea and psoriasis, help regulate vitamin D production, and are also involved in the production of extracellular matrix proteins such as hyaluronic acid enzymes that are important for maintaining the cutaneous barrier, such as sphingomyelinase.

Staphyloccocus epidermidis is the main micro-organism in skin microbiota and consequently remains in place, even during aging. Harmless for keratinocytes, *S. epidermidis* produces AMPs (epidermin, Pep5, epilancin K7, phenol-soluble modulins (PSMs) and epicidin 280) that control *S. aureus* and Streptococcus populations, protecting the skin.

*S. epidermidis* synthesizes proteases, keratinases, lipases and nucleases that contribute to the physiological turnover of the skin and play a role in keeping skin healthy, protecting it from harmful opportunistic micro-organisms and fostering physiological mechanisms that renew the skin.

There are countless active ingredients available with suggestive direct or indirect effects on the microbiota, administered either topically or by mouth, such as acetic acid, diacetyl acid, lactic acid and hyaluronic acid, among others. Although microbiota components and their direct and indirect effects on the skin are known, widespread use of antibacterial agents persists, affecting both beneficial and harmful bacteria on the skin. The indiscriminate use thereof weakens the natural defenses of the skin, leaving it more vulnerable to harmful bacteria.

This is why new prebiotic and probiotic ingredients are still being researched.

Prebiotics are ingredients that foster beneficial bacteria, while probiotics are micro-organisms that interact with the natural skin biota and encourage it to produce its own defenses. A probiotic may also act as a selective antibacterial agent, producing substances that control the proliferation of harmful bacteria.

Several prior art publications indicate that prebiotic agents may selectively favor beneficial bacteria and are thus candidates for inclusion in cosmetic compositions intended for this purpose. Nevertheless, despite screening and suggesting possible prebiotic agents, the state of the art is limited in terms of providing effective prebiotic ingredients with proven in vitro efficacy and with mechanisms of action that are clearly explained, particularly for topical administration, which is why investigation is still lacking.

The prior art further discloses "Repair Smoothing Cream", Mintel, 24 July 2015; "Buriti & Vegetal Protein Restorative Hair Mask", Mintel, 4 December 2006; "Night Recovery Moisturizer for Combination Oily to Oily Skin", Mintel, 9 March 2016I "Rejuvenating Cream", Mintel, 15 April 2016I "Regenerative Night Cream", Mintel, 28 October 2015; GB 498543 A which concerns a prebiotic powder compositions and uses thereof; and WO 2015/031971 A2 which concerns a composition comprising guacatonga extract and aroeira extract, a use thereof, and a method for preventing and/or treating signals caused by skin aging.

Consequently, the need persists for cosmetic compositions with proven prebiotic effects on the skin, particularly for topical administration.

### BRIEF DESCRIPTION OF THE FIGURES

Figure 1 presents compared assay results with different candidate prebiotic ingredients, showing the prebiotic effects of trehalose and babassu palm starch.
Figure 2 presents compared assay results with different candidate prebiotic ingredients, showing the prebiotic effects of crabwood *(andiroba),* Brazilnut and moriche (*buriti*) palm oils.
Figure 3 presents compared assay results with different candidate prebiotic ingredients, showing the prebiotic effects of wild sage *(guagatonga)* extract.
Figures 4A-D to 10A-D show the effects of trehalose, babassu palm starch (LIMS5345), crabwood *(andiroba)* oil (LIMS16648) and moriche (*buriti*) palm oil (SAP50002446) on elastin, GAG, IL-6, IL-8 and IL-10 syntheses, MMP-1 production and cell proliferation rates in human fibroblast cultures. The data indicate the mean ± the standard deviation for different independent experiments. **P<0.01, compared to the Baseline Control group (ANOVA, Dunnet).
Figures 11A and 11B show skin protection results for *S*. *epidermidis* against *S*. *aureus* - preventive effect.
Figures 12A and 12B show skin protection results for *S*. *epidermidis* against *S*. *aureus* - preventive effect.

### DESCRIPTION OF THE INVENTION

The invention provides a cosmetic non-therapeutic use according to claim 1. Further preferred features are defined in the dependent claims.

Thus, in an initial aspect, the present invention refers to prebiotic cosmetic compositions comprising at least one ingredient with prebiotic activity selected from sugars and oils for the treatment of signs of aging on the skin, fostering well-balanced skin, nourishing beneficial micro-organisms in the skin biota, protecting it from harsh environmental aspects, strengthening it, reducing sensitivity and irritations, and improving its defense system, with proven in vitro efficacy.

The sugar according to this invention is selected from trehalose and/or babassu palm starch, the oils are selected from crabwood *(andiroba),* moriche (*buriti*) palm, Brazilnut and wild sage *(guagatonga)* extract.

The prebiotic cosmetic compositions addressed by this invention may be presented in different cosmetic forms, including but not limited to, emulsions, gels, powders and sticks, among others, including carriers that are cosmetically suitable for the selected cosmetic form.

The prebiotic cosmetic compositions addressed by this invention are intended for topical administration and foster the proliferation of *S*. *epidermidis,* no increase in harmful micro-organisms

Surprisingly, a prebiotic effect was noted, caused by the active ingredients in the topical prebiotic cosmetic compositions addressed by this invention, whereby not only is it efficient for reducing harmful skin bacteria, fostering beneficial skin bacteria and ensuring evenly-balanced microbiota, it also leads to an even skin balance and the nutrition thereof, stimulating cell turnover, cell renewal, tissue renewal, cutaneous integrity and natural skin defense/protection, resulting in advantageous cosmetic effects such as enhanced skin firmness and elasticity, increase in the skin structuring matrix, strengthening skin architecture and restoring skin density, in addition to providing protection against enzymes that degrade the skin structuring matrix, protection against microinflammation, prevention of microdamage, protection against external aggression, soothing effects and more apparent effects on firmness.

Due to these and other effects noted, the topical cosmetic compositions addressed by this invention are used as anti-aging agents.

In another aspect, the topical prebiotic cosmetic compositions according to this invention present an anti-aging effect, acting directly on cell proliferation, skin protection and sensitivity reduction (through acting on IL-6, IL-8, IL-10 and PGE2 synthesis) together with firmness and elasticity (through acting on collagen and elastin; glycosaminoglycans and metalloproteinase modulation).

The increase in IL-6 and PGE2 reduces the responsiveness of innate immunity cells to external stimuli, such as variations temperature, moisture and UV radiation. The increase in IL-10 modulates the responsiveness of lymphocytes to stimuli such as inorganic particles and other micro-organisms. The reduction in IL-8 lowers the inflammatory cascade activation threshold without adversely affecting skin protection. In turn, increased collagen, elastin and glycosaminoglycans (GAGs) lead to greater firmness and elasticity for the dermis. Metalloproteinase modulation 1 (MMP-1) regulates collagen degradation and ensures skin physiology maintenance.

Particularly, babassu palm starch nourishes the skin, as this is a glucose dimer, protecting it by increasing the S. *epidermidis* population that produces AMPs and modulates inflammatory cytokines.

In addition to the benefits listed above, trehalose and moriche *(buriti)* palm oil also renew the skin by increasing the proliferation of fibroblasts and increasing skin hydration through increased absorption and retention of water by the skin, in addition to enhanced skin elasticity through elastin synthesis, together with odor control through not stimulating the proliferation of Corynebacteria.

The topical prebiotic cosmetic compositions addressed by this invention may be presented in any suitable cosmetic for application to the face or body for treatment or cleaning purposes with no constraints, as known to a person skilled in the art, including emulsions, solutions, powders, gels, pastes and toilet soaps, among others.

Cosmetically acceptable carriers may be selected from compounds known at the state of the art. Without imposing any constraints, they may consist of emollient, antioxidant, moisturizing, emulsifying and surfactant agents, texture or viscosity modifiers, preservatives, chelating, stabilizing and solubilizing agents, lubricants, thickeners, dispersants, and other cosmetically acceptable carriers. In another aspect, this invention also encompasses use of the cosmetic compositions addressed by this invention simultaneously as a prebiotic, selective bacteriostatic and skin anti-aging agent, together with the use of the active ingredients described herein, either alone or in combination, for the preparation of cosmetic compositions that are simultaneously pre-biotic, selective bacteriostatic and skin anti-aging agents.

The following examples illustrate this invention, without imposing any constraints thereon.

### EXAMPLES

### ASSESSMENT OF EFFECTS

When a concentration of 10^4 UFC / ml was reached, the micro-organisms were transferred to a minimal carbon support medium that provided the conditions required for micro-organism replication.

Each active ingredient in the compositions addressed by this invention was added to the culture medium and cultivated. Should any increased count be noted, the ingredient would be considered as having prebiotic activity.

The results of each assay were submitted to statistical analysis. The active ingredients used in the prebiotic cosmetic compositions addressed by this invention were tested on different micro-organisms.

The beneficial micro-organisms assayed were: *Staphylococcus epidermidis, Staphylococcus xylosus, Staphylococcus warneri, Staphylococcus captis* and *Corynebacterium xerosis.*

The undesirable micro-organisms assayed were: *Staphylococcus aureus, Corynebacterium striatum, Corynebacterium jeikeium, Propioniumbacteria acnes* and *Candida albicans.*

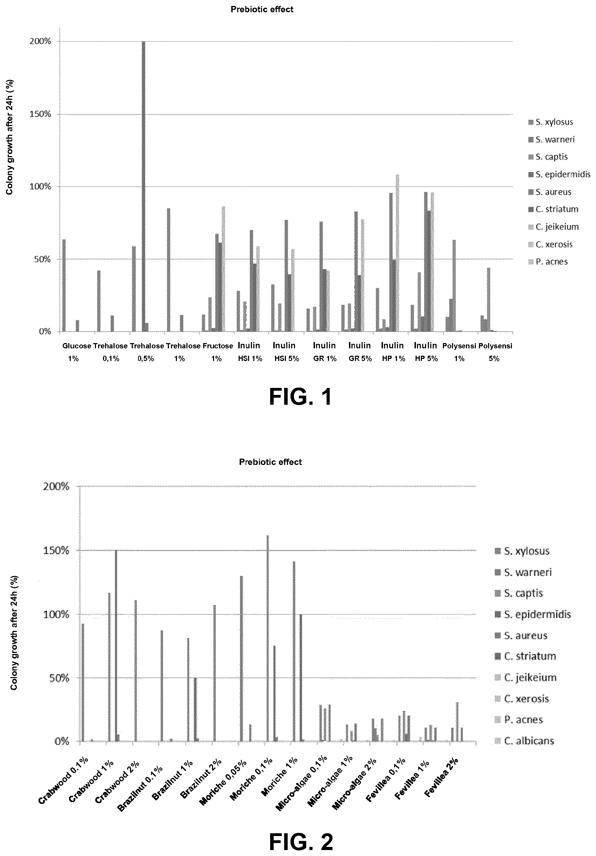

It was noted that inulin, which is widely used as a prebiotic in the food industry, did not present selectivity for any of the tested skin-dwelling micro-organisms, as shown in Figure 1.

This Figure also shows that trehalose, which is a cosmetic ingredient used for hydration, selectively favored the growth of *S*. *xylosus* and S. *epidermidis* (0.5%). This difference indicates differences in the microbial mechanism, with *S. epidermidis* being more sensitive to variations in the concentration of this active ingredient. Used as a texture modifier, babassu palm starch *(polisensi)* selectively favored *S. xylosus, S. warneri* and *S*. *captis.*

Figure 2 shows that crabwood *(andiroba),* Brazilnut and moriche *(buriti)* palm oils presented prebiotic activity, selectively modifying beneficial skin bacteria.

Crabwood *(andiroba)* oil fostered the growth of *S*. *xylosus* at all tested concentrations, and *S. epidermidis* at a concentration of 1%.

Moriche *(buriti)* palm oil fostered the growth of *S*. *xylosus* at all tested concentrations, and *S. epidermidis* as from 0.1%. The variation noted for *S*. *xylosus* falls within the margin of error for the test (20%).

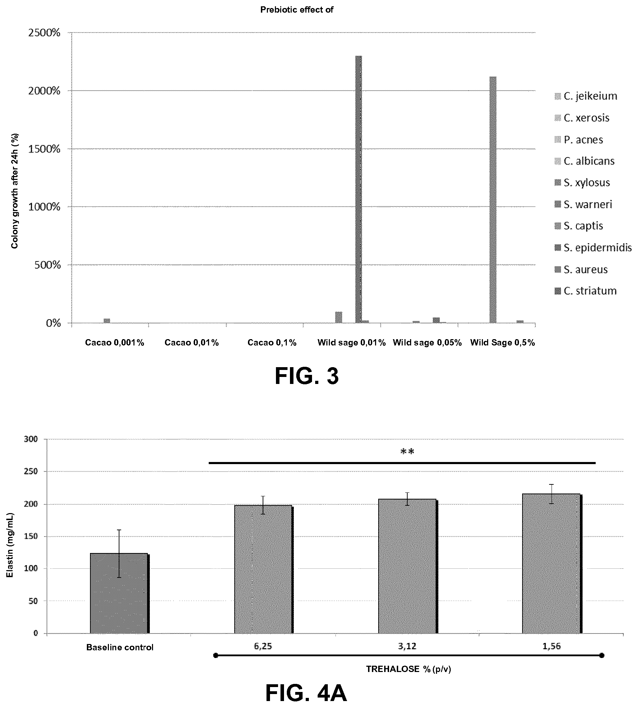

Figure 3 shows that wild sage (*guaçatonga*) extract fostered the growth of *S*. *epidermidis* at a concentration of 0.01% and *S. xylosus* at a concentration of 0.5%. This difference suggests that metabolic variations between the micro-organisms are sensitive to increased quantities of components in the extract.

In order to demonstrate the simultaneous effects described here, the active ingredients listed herein were tested against the anti-age mark parameters under assessment, including cell proliferation, IL-6 synthesis, IL-8, IL-10 and PGE2 synthesis, elastin; GAGs and MMP-1.

The results are presented in Table 1 below.

**TABLE 1. RESULTS FOR THE ASSESSED ANTI-AGE MARK ACTIVITY PARAMETERS (Feville oil is a reference oil )**

| | **Fibroblast proliferation** | **IL-8** | **IL-6** | **IL-10** | **PGE₂** | **Elastin** | **GAGs** | **MMP-1** |
|---|---|---|---|---|---|---|---|---|
| Babassu palm starch (0.01% - 0.05%) | - | Reduction of 13% (0.02%) | - | Increase of 65% | Reduction of 30% (0.02%) | Increase of 60% (0.02%) | Reduction of 40% (0.02%) | - |
| Trehalose (0.1% - 1%) | Increase of 33% | - | - | - | - | - | - | - |
| Moriche *(buriti)* palm oil (0.07% - 0.3%) | Dose-Dependent Increase of 47% to 103%, (0.02% - 0.07%) | - | Reduction of 33% (0.07%) | Reduction of 63% (0.02% - 0.07%) | - | Dose-Dependent Increase of 70% to 80% | Reduction of 42% (0.07%) | Reduction of 13% (0.02%) |
| Feville oil at (0.01% - 0.4%) | Increase of 20% (0.01% - 0.02%) | Reduction of 25% (0.04%) | Reduction of 73% to 37% (0.04% - 0.01%) | Reduction of 58% (0.04% - 0.01%) | Reduction of 36% to 60% (0.04% - 0.01%) | Increase of 60% to 36% (0.04% - 0.01%) | Increase of 46% (0.02%) | - |

Tests conducted under the aegis of this invention showed that the extrapolation of the activities of intestinal prebiotics to the skin did not prove true, stressing that each system is endowed with micro-organisms that are closely adapted thereto, with specific needs. Furthermore, it was noted that *S*. *epidermidis* and *S. xylosus* remained the main skin micro-organisms, even in the course of aging.

The literature shows that uncontrolled proliferation of *S*. *epidermidis* and *S. xylosus* leads to infectious conditions that are similar to those caused by *S. aureus.*

The prebiotic activity noted during the *in vitro* tests conducted for this invention may be facilitated *in vivo,* particularly for moriche *(buriti)* palm oil, due to the permeation facilitating activity of oleic acid (77% of the fatty composition of moriche *(buriti)* palm oil), which increases its availability to micro-organisms found at all skin levels.

Figures 4A-D to 10A-D show the effects of trehalose on elastin, GAG, IL-6, IL-8 and IL-10 syntheses, MMP-1 production and cell proliferation rates in human fibroblast cultures.

Figure 4A shows that trehalose increased elastin synthesis human fibroblast cultures, when applied at concentrations of 6.25%; 3.12 % and 1.56% (w/v) at 60%, 68% and 75%, respectively, compared to the Baseline Control group. Human fibroblast cultures were incubated for 48 hours with the product.

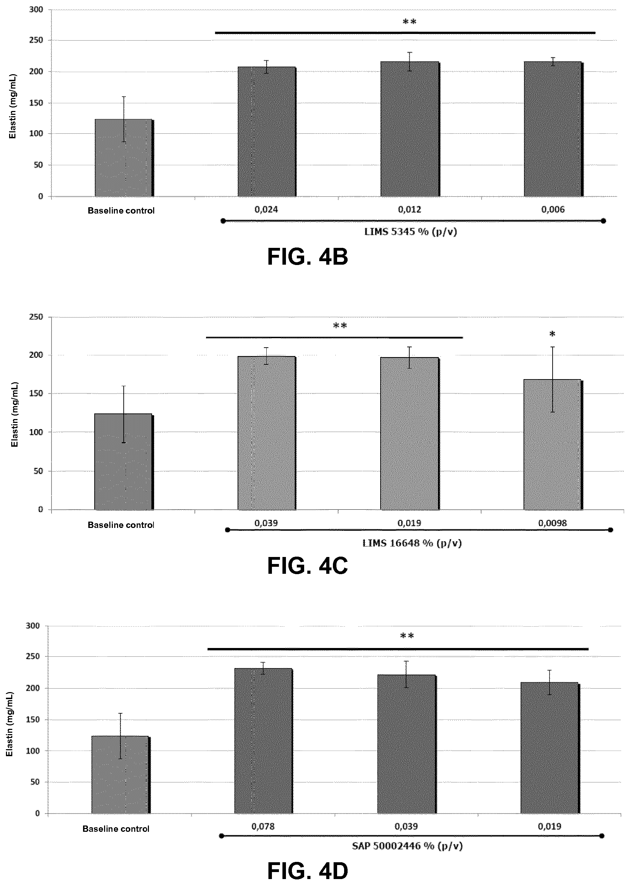

Figure 4B shows that babassu palm starch increased elastin synthesis in human fibroblast cultures when applied at concentrations of 0.024%; 0.012% and 0.006% (w/v) at 68%, 75% and 75%, respectively, compared to the Baseline Control group. Human fibroblast cultures were incubated for 48 hours with the product.

Figure 4C shows that crabwood *(andiroba)* oil increased elastin synthesis in human fibroblast cultures when applied at concentrations of 0.039%; 0.019% and 0.0098% (w/v) at 61.59% and 36%, respectively, compared to the Baseline Control group. Human fibroblast cultures were incubated for 48 hours with the product.

Figure 4D shows that moriche *(buriti)* palm oil increased elastin synthesis in human fibroblast cultures when applied at concentrations of 0.078%; 0.039% and 0.019% (w/v) at 88%, 80% and 70%, respectively, compared to the Baseline Control group. Human fibroblast cultures were incubated for 48 hours with the product.

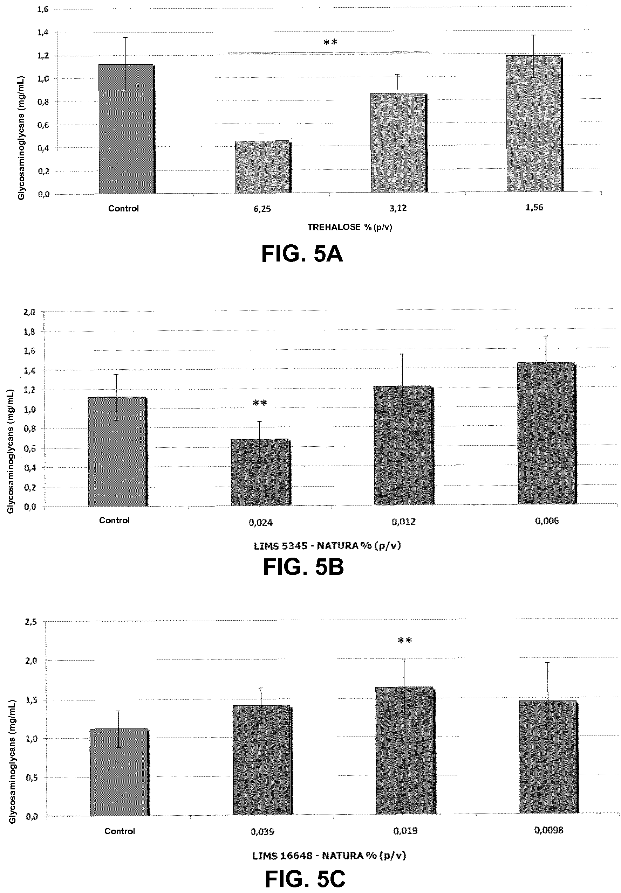

Figure 5A shows that trehalose significantly lowered GAG levels by 59% and 23%, when applied at concentrations of 6.25% and 3.12% (w/v), respectively, in a human fibroblast culture, for a period of 72 hours.

Figure 5B shows that babassu palm starch significantly lowered GAG levels when applied at a concentration of 0.024% (w/v) at up to 40% in a human fibroblast culture, for a period of 72 hours.

Figure 5C shows that crabwood *(andiroba)* oil proved able to increase GAG levels by 26.46% and 29%, when applied at concentrations of 0.039%, 0.019% and 0.0098% (w/v) in a human fibroblast culture, for a period of 72 hours, being statistically significant at a concentration of 0.019%.

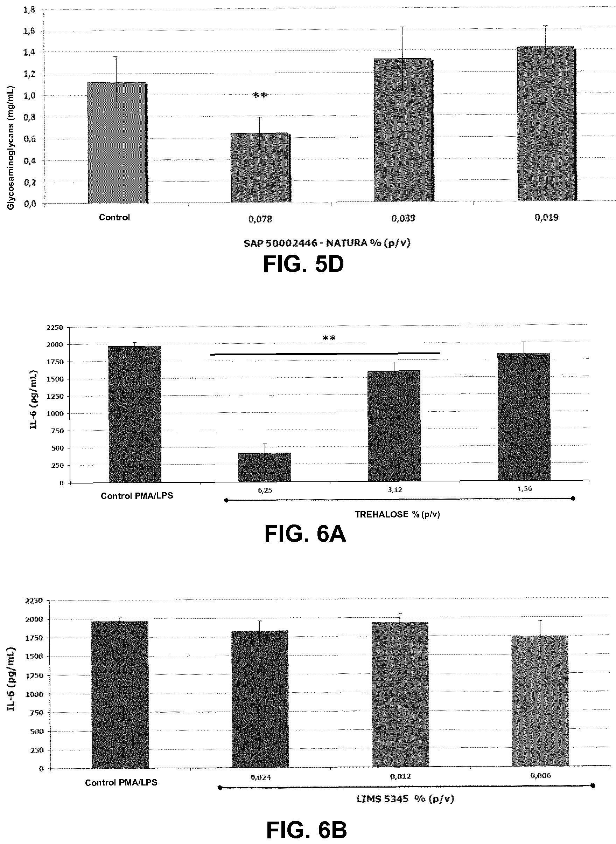

Figure 5D shows that moriche *(buriti)* palm oil significantly lowered GAG levels when applied at a concentration of 0.078% (w/v) at up to 42% in a human fibroblast culture, for a period of 72 hours.

Figure 6A shows that trehalose, when applied to cell cultures at concentrations of 6.25% and 3.12% (w/v), resulted in a drop of 78% and 19%, respectively for IL-6 synthesis in human fibroblast cultures stimulated with LPS, in terms of PMA/LPS Control.

Figure 6B shows that babassu palm starch when applied to cell cultures at concentrations of 0.024%; 0.012% and 0.006% (w/v), did not cause any alteration to IL-6 synthesis in human fibroblast cultures stimulated with PMA/LPS, in terms of PMA/LPS Control.

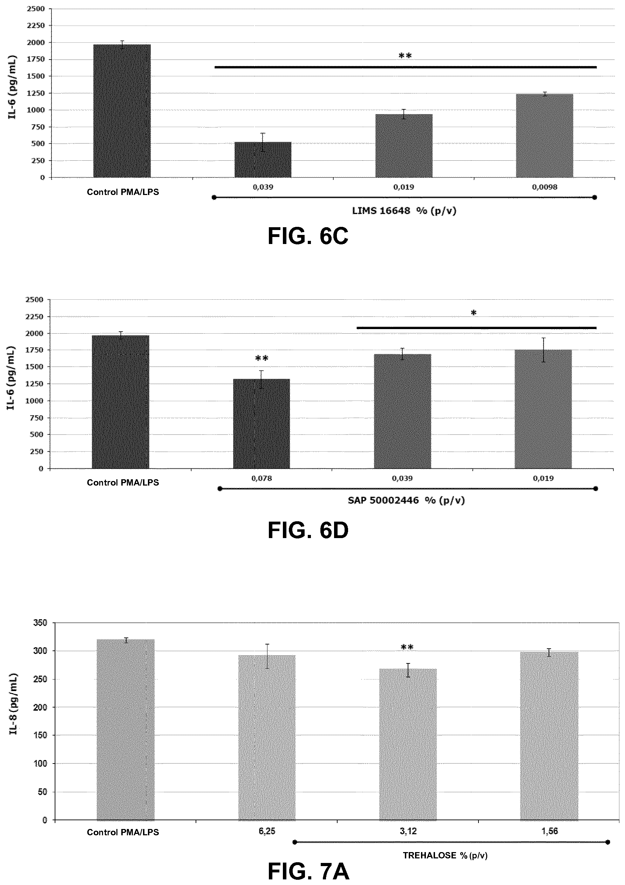

Figure 6C shows that crabwood *(andiroba)* oil when applied to cell cultures at concentrations of 0.039%; 0.019% and 0.0098% (w/v), resulted in a drop of 73.52% and 37%, respectively for IL-6 synthesis in human fibroblast cultures stimulated with PMA/LPS, in terms of PMA/LPS Control.

Figure 6D shows that moriche (*buriti*) palm oil when applied to cell cultures at concentrations of 0.078%; 0.039% and 0.019% (w/v), resulted in a drop of 33%, 14% and 10%, respectively for IL-6 synthesis in human fibroblast cultures stimulated with PMA/LPS, in terms of PMA/LPS Control.

Figure 7A shows that trehalose, when applied to cell cultures at a concentration of 3.12% (w/v), caused a statistically significant reduction of 16% for IL-8 synthesis in human fibroblast cultures stimulated with LPS, in terms of the LPS Control.

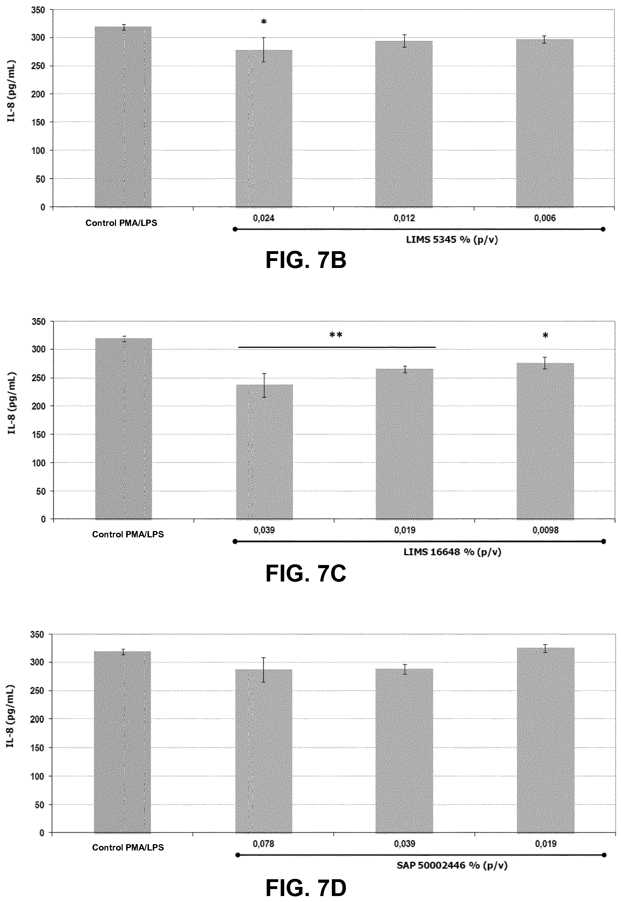

Figure 7B shows that babassu palm starch when applied to cell cultures at a concentration of 0.024% (w/v), caused a statistically significant reduction of 13% for IL-8 synthesis in human fibroblast cultures stimulated with LPS, in terms of the LPS Control.

Figure 7C shows that crabwood *(andiroba)* oil when applied to cell cultures at concentrations of 0.039%; 0.019% and 0.0098% (w/v), caused a statistically significant reduction of 25%, 17% and 13%, respectively in the synthesis of IL-8 in human fibroblast cultures stimulated with LPS, in terms of the LPS Control.

Figure 7D shows that moriche (*buriti*) palm oil when applied to cell cultures at concentrations of 0.039%; 0.019% and 0.0098% (w/v), did not cause any statistically significant alteration to IL-8 synthesis in human fibroblast cultures stimulated with LPS, in terms of the LPS Control.

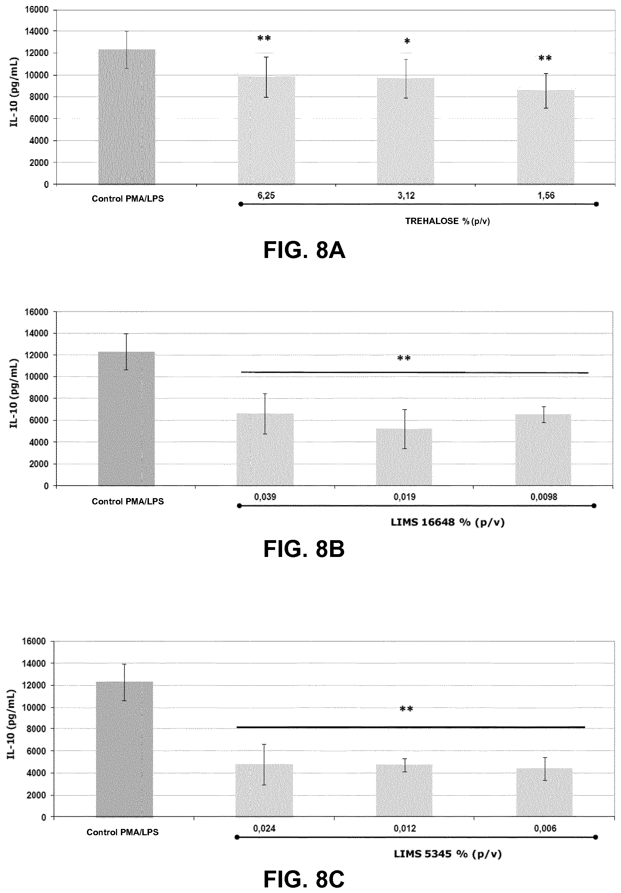

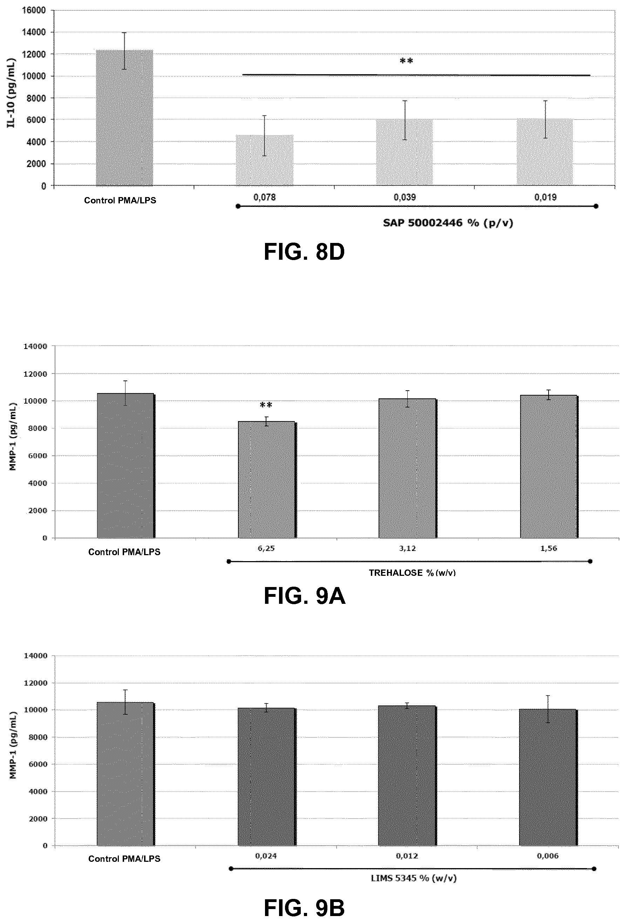

Figure 8 shows that trehalose, when applied to cell cultures, caused reductions of up to 31% for IL-10 synthesis in human fibroblast cultures stimulated with PMA/LPS, in terms of PMA/LPS Control.

Figure 8B shows that babassu palm starch when applied to cell cultures, caused reductions of up to 65% for IL-10 synthesis in human fibroblast cultures stimulated with PMA/LPS, in terms of PMA/LPS Control.

Figure 8C shows that crabwood *(andiroba)* oil when applied to cell cultures at caused reductions of up to 58% for IL-10 synthesis in human fibroblast cultures stimulated with PMA/LPS, in terms of PMA/LPS Control.

Figure 8D shows that moriche (*buriti*) palm oil when applied to cell cultures, caused reductions of up to 63% for IL-10 synthesis in human fibroblast cultures stimulated with PMA/LPS, in terms of PMA/LPS Control.

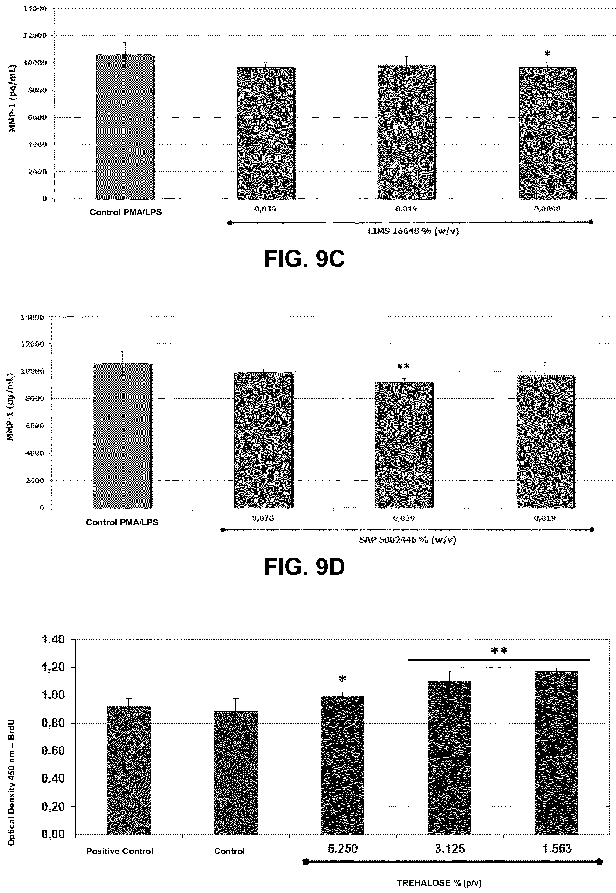

Figure 9 shows that trehalose, when applied to cell cultures, at a concentration of 6.25% (p/v), caused a 20% reduction in MMP-1 synthesis in human fibroblast cultures stimulated with LPS, in terms of PMA/LPS Control.

Figure 9B shows that babassu palm starch when applied to cell cultures at concentrations of 0.024%; 0.012% and 0.006% (w/v), did not cause any alteration to MMP-1 synthesis in human fibroblast cultures stimulated with LPS, in terms of PMA/LPS Control.

Figure 9C shows that crabwood *(andiroba)* oil when applied to cell cultures at a concentration of 0.0098% (w/v), resulted in a drop of 9% for MMP-1 synthesis in human fibroblast cultures stimulated with LPS, in terms of PMA/LPS Control.

Figure 9D shows that moriche (*buriti*) palm oil when applied to cell cultures at a concentration of 0.039% (w/v), resulted in a drop of 13% for MMP-1 synthesis in human fibroblast cultures stimulated with LPS, in terms of PMA/LPS Control.

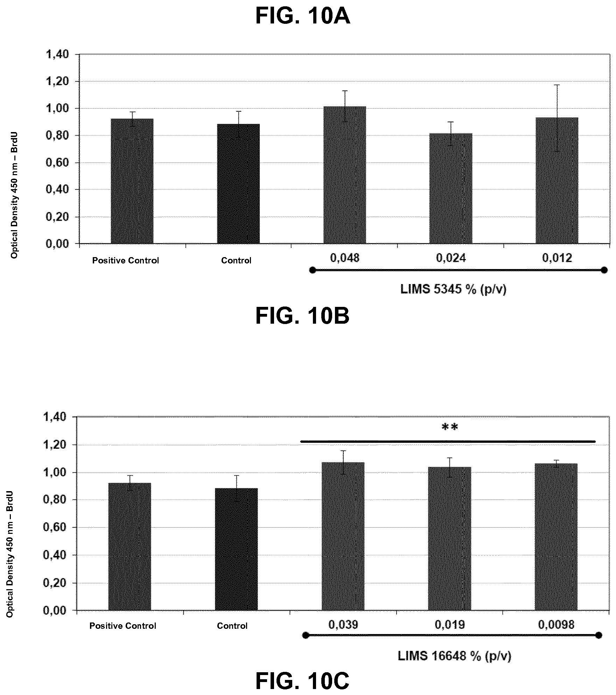

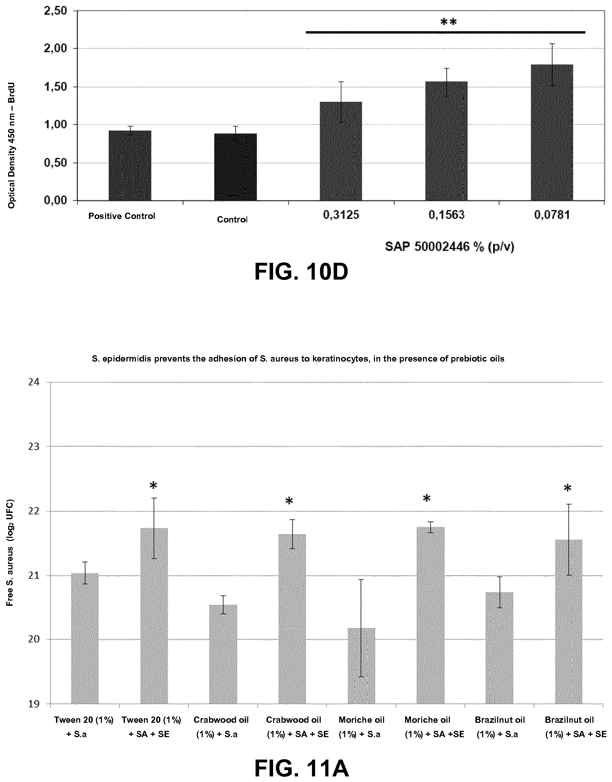

Figure 10 shows that trehalose proved able to increase proliferation during the length of time assessed at concentrations of 6.250%; 3.125% and 1,563% (w/v) in 12.25% and 33%, respectively, compared to the Control group for the same length of time.

Figure 10B shows that babassu palm starch proved unable to alter proliferation during the length of time assessed at the tested concentrations of 0.048%, 0.024% and 0.012% (w/v) compared to the Control group for the same length of time.

Figure 10C shows that crabwood *(andiroba)* oil proved able to increase proliferation during the length of time assessed at the tested concentrations of 0.039%; 0.019% and 0.0098% (w/v) at 21%, 17% and 20%, respectively, compared to the Control group for the same length of time.

Figure 10D shows that moriche *(buriti)* palm oil proved able to increase proliferation during the length of time assessed at concentrations of 0.3125%, 0.1563% and 0.0781% (w/v) at 47.77% and 103%, respectively, compared to the Control group for the same length of time.

As a positive assay control, human fibroblasts were cultivated in a culture medium supplemented with 2% of fetal bovine serum.

The protective effect of *S*. *epidermidis* against *S*. *aureus* in keratinocytes (HaCaT) in a culture was also assessed.

To do so, Step I consisted of defining the bacteria concentration through cell feasibility; Step II consisted of prebiotic dilution; Step III consisted of a preventive protocol (*S. epidermidis* prevents the adhesion of *S*. *aureus*); and Step IV consisted of a competition protocol (*S*. *epidermidis* prevents the adhesion of *S*. *aureus).*

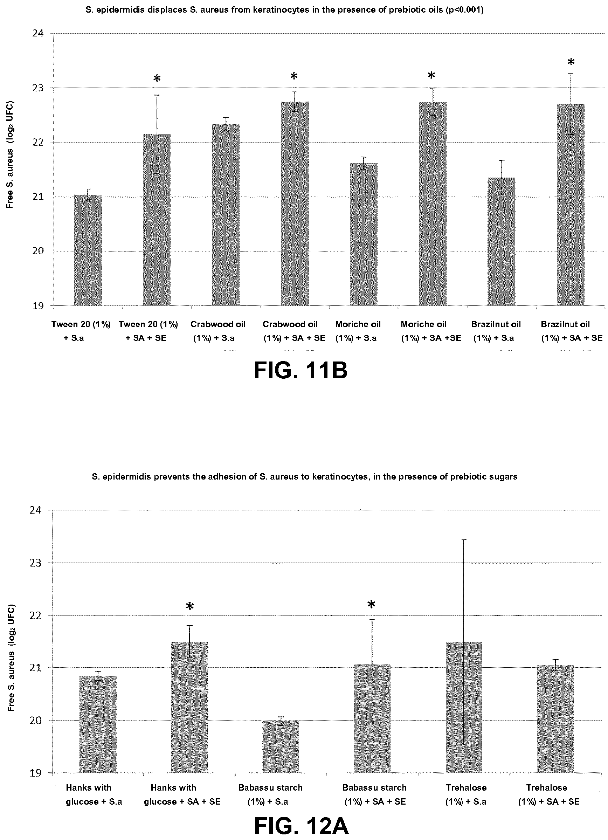

Figures 11A and 11B show skin protection results for *S. epidermidis* against *S*. *aureus* - preventive effect.

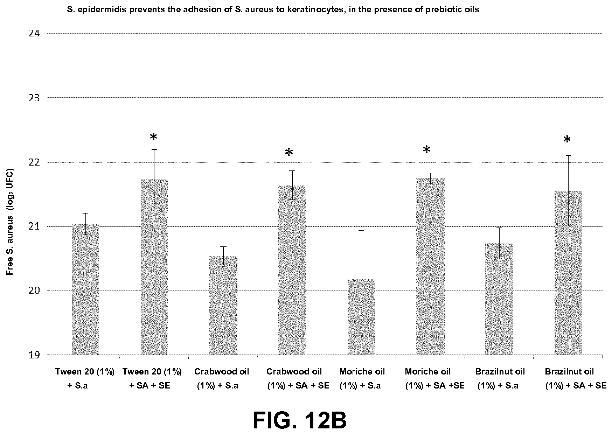

Figures 12A and 12B show skin protection results for *S*. *epidermidis* against *S*. *aureus* - preventive effect.

Together, these results show that the prebiotic effect caused by the active ingredients in the topical prebiotic cosmetic compositions according to the present invention is not only efficient for reducing harmful skin bacteria and fostering beneficial skin bacteria, ensuring an evenly-balanced microbiota, but also ensures a well-balanced skin that is nourished, stimulating cell turnover, cell renewal, tissue renewal, cutaneous integrity and natural skin defense/protection, resulting in advantageous cosmetic effects such as enhanced skin firmness and elasticity, increase in the skin structuring matrix, strengthening skin architecture and restoring skin density, in addition to providing protection against enzymes that degrade the skin structuring matrix, protection against microinflammation, prevention of microdamage, protection against external aggression, soothing effects

## Claims

1. A cosmetic non-therapeutic use of at least one sugar or oil as a prebiotic with a cosmetically acceptable carrier, wherein the sugar is selected from trehalose or babassu palm starch, and the oil is selected from crabwood *(andiroba),* moriche (*buriti*) palm, Brazilnut, and wild sage *(guagatonga)* extract.

2. The cosmetic non-therapeutic use of claim 1, wherein the use as a prebiotic is selective to *Staphylococcus epidermis, Staphylococcus xylosus, Staphylococcus warneri*, *Staphylococcus captis, Corynebacterium xerosis* or a combination thereof, preferably wherein the use as a prebiotic is selective to *Staphylococcus epidermis.*

3. The cosmetic non-therapeutic use of any preceding claim, wherein said use is by topical application.

## Patentansprüche

1. Kosmetische, nicht therapeutische Verwendung von mindestens einem Zucker oder Öl als Präbiotikum mit einem kosmetisch akzeptablen Träger, wobei der Zucker aus Trehalose oder Babassupalmstärke ausgewählt wird und das Öl aus Extrakt von *Andirobaholz,* Moriche- (*Buriti*-) Palme, Paranuss und wildem Salbei (*Guaçatonga*) ausgewählt wird.

2. Kosmetische, nicht therapeutische Verwendung nach Anspruch 1, wobei die Verwendung als Präbiotikum selektiv gegen *Staphylococcus epidermis, Staphylococcus xylosus, Staphylococcus warneri, Staphylococcus captis, Corynebacterium xerosis* oder eine Kombination davon ist, bevorzugt, wobei die Verwendung als Präbiotikum selektiv gegen *Staphylococcus epidermis* ist.

3. Kosmetische, nicht therapeutische Verwendung nach einem vorstehenden Anspruch, wobei die Verwendung durch topische Anwendung erfolgt.

## Revendications

1. Utilisation non thérapeutique cosmétique d'au moins un sucre ou une huile en tant que prébiotique avec un support cosmétiquement acceptable, dans laquelle le sucre est sélectionné parmi le tréhalose ou la fécule de palmier babassu, et l'huile est sélectionnée parmi l'extrait de carapa (*andiroba*), de palmier moriche (*buriti*)*,* de noix de Brésil, et de sauge sauvage (*guaçatonga*).

2. Utilisation non thérapeutique cosmétique selon la revendication 1, dans laquelle l'utilisation en tant que prébiotique est sélective pour *Staphylococcus epidermidis, Staphylococcus xylosus, Staphylococcus warneri, Staphylococcus capitis, Corynebacterium xerosis* ou une combinaison de celles-ci, de préférence dans laquelle l'utilisation en tant que prébiotique est sélective pour *Staphylococcus epidermidis.*

3. Utilisation non thérapeutique cosmétique selon une quelconque revendication précédente, dans laquelle ladite utilisation s'effectue par application topique.
